# EUROPÄISCHE PATENTSCHRIFT

(11) **EP 0 516 635 B1**
(45) Veröffentlichungstag und Bekanntmachung des Hinweises auf die Patenterteilung: **25.09.1996**
(21) Anmeldenummer: 91902596.5
(22) Anmeldetag: 21.12.1990
(51) Int. Cl.: B05B 7/14, B05B 11/06

(54) **AUSTRAGVORRICHTUNG FÜR MEDIEN**
DISPENSER FOR MEDIA
DISTRIBUTEUR DE MILIEUX

(30) Priorität: 22.02.1990 DE 4005527
(43) Veröffentlichungstag der Anmeldung: 09.12.1992
(73) Patentinhaber: Ing. Erich Pfeiffer GmbH, 78315 Radolfzell (DE)
(72) Erfinder: FUCHS, Karl-Heinz, D-78315 Radolfzell (DE)
(74) Vertreter: Patentanwälte Ruff, Beier, Schöndorf und Mütschele
(86) Internationale Anmeldenummer: EP9002279
(87) Internationale Veröffentlichungsnummer: WO9112895

(56) Entgegenhaltungen:
- DE-C- 94 040
- FR-A- 2 454 331
- US-A- 1 752 956
- US-A- 1 911 972
- US-A- 3 045 388
- US-A- 3 907 206

## Beschreibung

Die Erfindung betrifft eine Austragvorrichtung nach dem Oberbegriff des Patentanspruches 1.

Sie soll für Medien, insbesondere für ein einziges Medium, gesondert gespeicherte, gleiche oder ungleiche, fließfähige Medien von ggf. unterschiedlichen Aggregatzuständen geeignet sein und weist mindestens eine handbetätigbare Pumpe, wenigstens eine Pumpkammer, mindestens einen zu einer Austragöffnung führenden Auslaßkanal sowie wenigstens eine von der jeweiligen Pumpkammer gesonderte Medienkammer auf, welche in wenigstens einer Stellung der Austragvorrichtung mit der Austragöffnung und/oder der Pumpkammer leitungsverbunden ist.

Durch die US-A-1 911 972 und die US-A-3 045 388 sind Austragvorrichtungen bekanntgeworden, bei welchen zwar von einer Luftpumpe direkt in eine Medienkammer und unter Aufnahme von Medium dann aus dieser herausgefördert wird, jedoch durchströmt hier die Luft die Medienkammer in völlig unkontrollierter Weise gegenläufig und außermittig, so daß die Sättigung des Luftstromes mit Medium willkürliche Zufallswerte annimmt.

Durch die US-A-3 907 206 ist eine weitere Austragvorrichtung bekanntgeworden, bei welcher die Medienkammer nicht von dem Förderluftstrom der Pumpe durchströmt wird, was die Förderung schwerfließender Medien erschwert.

Der Erfindung liegt die Aufgabe zugrunde, Nachteile bekannter Lösungen zu vermeiden und insbesondere eine Austragvorrichtung der genannten Art zu schaffen, mit welcher ein ggf. vorgespeichertes Medienquantum unter Zuhilfenahme eines weiteren Förder- bzw. Trägermediums ausgetragen werden kann.

Erfindungsgemäß sind die Merkmale des Patentanspruches 1 vorgesehen.

Vorteilhaft ist eine in ihrer Ausgangsstellung im wesentlichen dicht geschlossene Medienkammer vorgesehen, in welcher eine genau dosierte Medienmenge auch über lange Lagerzeiten geschützt und frisch gehalten gespeichert werden kann und die zum Austrag des Mediums so beeinflußt werden kann, daß sie in Leitungsverbindung mit der Pumpe steht und bei deren Betätigung von dem gepumpten Medium durchströmt wird. Zweckmäßig wird das Medium simultan aus der Medienkammer ausgetragen und ggf. in einem einzigen Pumpstoß bis zur Austragöffnung gefördert. Handelt es sich um ein pulverförmiges Medium, so wird es zweckmäßig mit einem gasförmigen Pumpmedium fluidisiert und von einem Ende der Medienkammer zum gegenüberliegenden Ende hinausgedrückt.

Das Pumpmedium kann in einem ersten, an die Ausgangsstellung anschließenden Teilhub vorgespannt und dann erst die Leitungsverbindung zur Medienkammer geöffnet werden. Zweckmäßig liegt die Medienkammer nicht auf der Saug-, sondern auf der Druckseite der Pumpe, so daß das Medium die Pumpkammer nicht durchströmt. Die Medienkammer kann durch geeignete Steuerung auch vor Beginn des Pumphubes am Einlaß und/oder am Auslaß durch eine geeignete Folgeschaltung geöffnet werden; muß zur Einleitung des Pumphubes eine federnde Rastung durch Aufbringen eines entsprechenden Betätigungsdruckes überwunden werden, so ergibt sich von selbst eine schlagartige Betätigung der Pumpe und dadurch ebenfalls ein Austrag des Mediums in einem einzigen Stoß. Zur Erzielung hoher Strömungsgeschwindigkeiten des Pumpmediums hat der Verbindungskanal zwischen der Pumpkammer und der Medienkammer zweckmäßig wesentlich kleinere Durchlaß-Querschnitte als eine oder beide Kammern, wobei die Durchlaßquerschnitte in Richtung zur Medienkammer abnehmen können.

Der Speicherraum der Medienkammer ist im Querschnitt zweckmäßig mit mindestens einer Unterbrechung bzw. mit mindestens einem innerhalb seiner Außen-Umfangsbegrenzung liegenden Füllstück versehen, so daß das gespeicherte Medium trotz großen Aufnahmevolumens nur verhältnismäßig kleine Vollquerschnitte bildet.

Vorteilhaft ist die Austragvorrichtung so ausgebildet, daß sie mit den bzw. drei Fingern einer einzigen Hand getragen und gleichzeitig sicher betätigt sowie z.B. nach Art einer Munddusche verwendet werden kann. Die Austragvorrichtung kann dabei als Einweg-Austragvorrichtung ausgebildet sein, die nach Leerung der Medienkammer nicht mehr gefüllt wird und daher als einfache Baueinheit ausgebildet sein kann, die die Pumpe, den Medienspeicher, Kanäle und ggf. Ventile bzw. Verschlüsse innerhalb eines Außengehäuses aufnimmt, das in Seitenansicht etwa T- bzw. Y-förmig sein kann. Ist die Austragvorrichtung, nämlich die Pumpkammer und die Medienkammer, dafür vorgesehen, nur in einem einzigen ganzen Pumphub in aufeinander folgenden Teilstrecken oder in einem Durchgang vollständig und nicht wieder füllbar entleert zu werden, so können beide Kammern in der Ausgangslage nach außen vollständig dicht geschlossen sein, und die Pumpkammer bedarf keinerlei Ventiles, zumindest keines Einlaßventiles. Eine Medienkammer kann einteilig mit der ggf. in einteiliger Bauweise einen Pumpenzylinder bildenden Pumpkammer ausgebildet sein, und/oder eine ggf. weitere Medienkammer kann an mindestens einem von der Pumpkammer gesonderten Bauteil vorgesehen sein, z.B. mit mindestens einer Begrenzung eine Baueinheit mit einem Pumpkolben und/oder mit mindestens einer Begrenzung eine ebenfalls ggf. einteilige Baueinheit mit einem Gehäuse bzw. Grundkörper der Austragvorrichtung, mit einem Auslaßkanal oder dgl. bilden.

Die Austragvorrichtung ist in Axialansicht zweckmäßig länglich gestreckt, so daß sie eine oder zwei gegenüberliegende und über verhältnismäßig schmale Abrollkanten ineinander übergehende Flachseiten bildet, auf denen sie ihre stabilste Lage hat, so daß sie bevorzugt die entsprechende liegende Lage einnimmt. Liegen in diesem Fall die Betätigungshandhaben quer zur Liegeebene frei zugänglich und voneinander abgekehrt sowie ggf. im Axialabstand voneinander, so kann die Austragvorrichtung leicht an ihren Handhaben gegriffen und mit derselben Greiflage der Finger auch betätigt werden. Zweckmäßig sind beide gegenüberliegenden Flachwandungen des Gehäuses von Daumen-Eingriffsöffnungen durchsetzt, die gemeinsam eine Aufnahme für einen Daumen bilden, so daß sämtliche Funktionsteile der Austragvorrichtung geschützt im wesentlichen vollständig innerhalb des Gehäuses liegen können. Sind die Daumenausschnitte der Austragvorrichtung, die dadurch an ihrem von der Austragöffnung entfernten Ende eine Gabel mit zwei im wesentlichen starren und etwa parallel frei ausragenden Gabelfingern bildet, verhältnismäßig eng, so kann die Austragvorrichtung, insbesondere nach der Betätigung, wie eine Klammer auf dem Finger bzw. Daumen getragen werden, was Handhabungen unmittelbar im Anschluß an den Austrag erleichtert.

Die genannten Teile der Pumpe und/oder der Medienkammer können, vorzugsweise als mindestens eine in sich geschlossene Baueinheit, über eine reine Steckverbindung an einem Grundkörper befestigt sein, der vorzugsweise das Außengehäuse bildet und im Innern einen vorstehenden Steckansatz aufweist, mit welchem die jeweilige Baueinheit, insbesondere etwa in Richtung der Pumpbetätigung, verbunden ist. Es können auch zwei kolbenartige Bauteile oder dgl. im Abstand zueinander an einem Schaft vorgesehen sein, der in einer gegenüber den Kolben engeren Bohrung geführt ist, so daß die zugehörige Baueinheit in beiden Verschieberichtungen anschlagbegrenzt ist. In einer Verschieberichtung kann dabei der zugehörige Kolben oder dgl. wenigstens teilweise durch das gespeicherte Medium anschlagbegrenzt sein.

Mindestens eine Öffnung der Medienkammer bzw. deren Auslaß und/oder Einlaß kann auch im wesentlichen ventilfrei lediglich durch einen anschließenden Labyrinthkanal oder dgl. verschlossen sein, durch den das gespeicherte Medium erst strömt, wenn es unter entsprechend hohem Betriebsdruck steht bzw. von einem Trägermedium aufgenommen worden ist.

Diese und weitere Merkmale von bevorzugten Weiterbildungen der Erfindung gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung verwirklicht sein und vorteilhafte Ausführungen darstellen können. Ausführungsbeispiele der Erfindung sind in den Zeichnungen dargestellt und werden im folgenden näher erläutert. In den Zeichnungen zeigen:
- Fig. 1: eine erfindungsgemäße Austragvorrichtung im Axialschnitt,
- Fig. 2: die Austragvorrichtung gemäß Fig. 1 in Ansicht von unten und
- Fig. 3: eine weitere Ausführungsform in einer Darstellung entsprechend Fig. 1.

Die Austragvorrichtung 1 weist einen hohlen Grundkörper 2 auf, der am Ende eines frei vorstehender Halses 3 einen Stutzen 4 und am davon abgekehrten Ende ein erweitertes, allseits über den Umfang des Halses 3 vorstehendes Gehäuse 5 bildet. In dem kappeförmigen, von einem abgeflachten Mantel begrenzten und am hinteren Ende offenen Gehäuse 5 und in dem Hals 3 ist eine Schubkolben-Pumpe 6 angeordnet. Sie weist einen einteiligen, napfförmigen, im wesentlichen zylindrischen Zylinder 7 mit darin gelagertem und in einer Ausgangsstellung überwindbar festgesetzten Kolben 8 auf, die eine Pumpkammer 9 begrenzen. Alle genannten Bauteile und Anordnungen liegen in einer gemeinsamen Mittelachse 10, wie auch ein Auslaßkanal 11, eine Medienkammer 12, eine Austragkammer 13, eine Auslaßöffnung 14, ein Einlaß 15, ein Einlaßventil 16, ein Auslaß 17, ein Auslaßventil 18, ein Tragansatz 19, ein Kolbenschaft 20, ein Verschlußkörper 21, ein Verschlußkörper 22 und/oder Kanalabschnitte 23, 24, 25. Sämtliche erläuterten Formen, Lagen und Größen können genau oder nur angenähert bzw. ungefähr oder von den Angaben abweichend vorgesehen sein.

Der Auslaßkanal 11 verbindet die Medienkammer 12 mit der Pumpkammer 9 nach Öffnung des Einlaßventiles 16 und wird im wesentlichen von der einteiligen Baueinheit aus Kolben 8 und Kolbenschaft 20 begrenzt. Die zylindrische Medienkammer 12 ist in dem rohrförmigen Tragansatz 19 vorgesehen, der im wesentlichen berührungsfrei innerhalb des Halses 3 und zum Teil im Gehäuse 5 liegt und mit seinem vorderen Ende einteilig am hinteren Ende des Stutzens 4 in den Hals 3 übergeht. Die Medienkammer 12 liegt unmittelbar benachbart zum hinteren Ende des Stutzens 4 und geht in diesen über einen trichterförmig erweiterten Übergangsabschnitt über. Die Außenweite sowie das Fassungsvolumen der Medienkammer 12 sind kleiner als diejenigen der Pumpkammer 9 und der vom Stutzen 4 begrenzten Auslaßkammer 13, welche gegenüber der Pumpkammer 9 größer ist. Das auf voller Weite offene vordere Ende der Auslaßkammer 13 bildet die Auslaßöffnung 14.

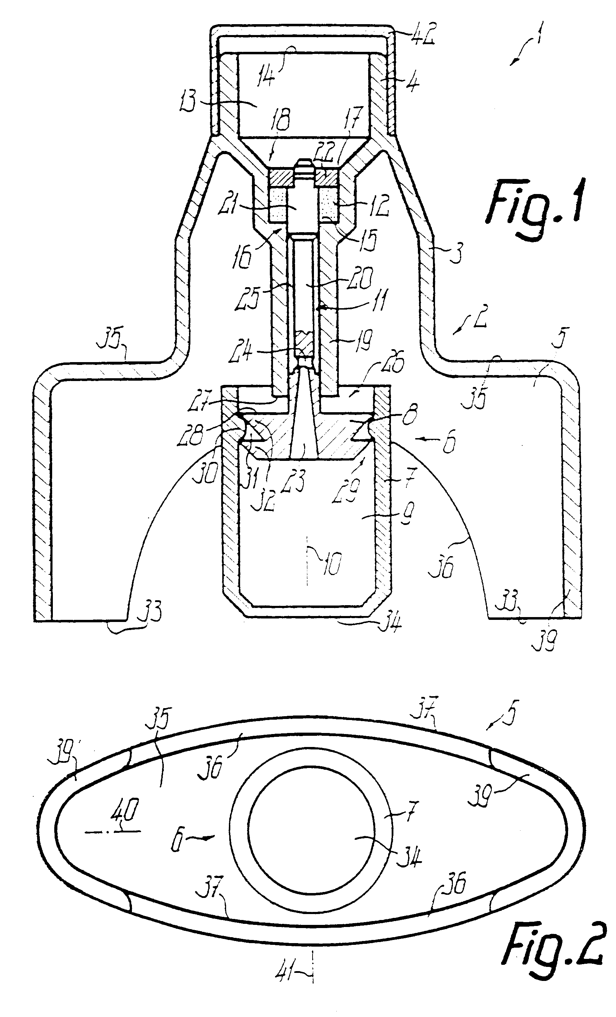

An das hintere Ende der Medienkammer 12 schließt ein im Querschnitt reduzierter Rohransatz des Tragansatzes 19 an, in dessen Bohrung der Kolbenschaft 20 abgedichtet, jedoch längsverschiebbar geführt ist. Der Kolbenschaft 20 bildet bzw. trägt zwei Ventil- bzw. Verschlußkörper 21, 22, mit welchen der am vorderen Ende des Auslaßkanales 11 liegende Einlaß 15 und der gegenüberliegende Auslaß 17 in der Ausgangsstellung gemäß Fig. 1 verschlossen und nach einer ersten Betätigungsphase geöffnet sind. Der Verschlußkörper 21 ist durch einen erweiterten Abschnitt des Kolbenschaftes 20 gebildet, dessen dahinter anschließender Schaftabschnitt mit dem Tragansatz 19 einen ringförmigen Kanalabschnitt 25 begrenzt. An das hintere Ende dieses Kanalabschnittes 25 schließt ein den Kolbenschaft 20 durchsetzender Querkanal 24 an, an den ein den Kolben 8 durchsetzender, in Strömungsrichtung spitzwinklig verjüngter Kanalabschnitt 23 anschließt, dessen hinteres Ende in der zugehörigen Kolbenstirnfläche mit der Pumpkammer 9 verbunden ist. Der andere Verschlußkörper 22 ist am vorderen Ende des Kolbenschaftes 20 bzw. Verschlußkörpers 21 angeordnet und ein Scheiben- bzw. Ringkörper, der mit seinem Außenumfang am zylindrischen Innenumfang der Medienkammer 12 abgedichtet gleitbar anliegt.

Zur vorangehenden Öffnung des Auslaßventiles 18 der Medienkammer 12 und selbsttätig sofort nachfolgendem Beginn des Pumpbetriebes der Pumpe 6 ist eine Folgeschaltung 26 vorgesehen. Die vordere Endfläche des Tragansatzes 19 bildet eine Mitnahmefläche 27, welcher eine Anschlagfläche 28 bzw. der zugehörigen Stirnfläche des Pumpkolbens 8 um einen Leerlaufweg gegenüberliegt, der gleich groß wie der Öffnungsweg des Auslaßventiles 18 ist. Wird der Zylinder 7 in Betätigungsrichtung bewegt, so nimmt er den Kolben 8 aufgrund dessen Hemmung gegenüber der Zylinderlaufbahn zunächst bis zur Anlage der Flächen 27, 28 mit, was zur gleichzeitigen Öffnung der Ventile 16, 18 führt. Bei weiterer Bewegung wird der Kolben 8 im Zylinder 7 in Pumprichtung bewegt, so daß aus der Pumpkammer 9 Luft durch den Auslaßkanal 11 mit beschleunigter Strömungsgeschwindigkeit über den Einlaß 15 in die Medienkammer 12 und von dort unter Mitnahme des gespeicherten Mediums durch den Auslaß 17 in die Verwirbelungs- bzw. Auslaßkammer 13 und dann durch die Auslaßöffnung 14 gelangt. In der Öffnungsstellung steht der Verschlußkörper 22 in der Auslaßkammer 13 dem Auslaß 17 als Prallteller gegenüber, wodurch eine Verwirbelung des Mediums beim Übertritt in die Auslaßkammer 13 erfolgt. Am Ende des Pumphubes liegt der Kolben 8 am Boden des Zylinders 7 an, der seinerseits zum größten Teil innerhalb des Halses 3 liegt.

Zur Lagesicherung von Zylinder 7 und Kolben 8 in der Ausgangsstellung gegeneinander ist eine Rasteinrichtung 29 vorgesehen. Diese weist am Innenumfang des Zylinders 7 und mit geringem Abstand von diesem offenen Ende einen ringwulstförmigen Rastnocken 30 auf, der in einer ringförmigen Rastnut 31 am Außenumfang des Kolbens 8 liegt. Die Rastnut 31 ist durch eine Zwischennut zwischen zwei axial benachbarten Kolbenlippen 32 gebildet, die einteilig mit dem Kolben 8 bzw. dem Kolbenschaft 20 ausgebildet sind und im Querschnitt ein spitzwinklig gezahntes Profil ergeben. Die ineinandergreifenden Rastglieder 30, 31 ergeben auch einen hermetisch dichten Verschluß der Pumpkammer 9 in der Ausgangsstellung. Durch entsprechend hohen Druck kann die Rastung überwunden werden, wobei jedoch zumindest eine Kolbenlippe vom Rastnocken nicht verformt wird, während eine benachbarte unter Aufrechterhaltung der dichten Anlage den Rastnocken 30 überspringt. Der Stellweg des Verschlußkörpers 22 ist wesentlich kleiner als seine Entfernung von der Auslaßöffnung 14, so daß er in jeder Stellung verhältnismäßig weit hinter dieser zurücksteht. In Ausgangsstellung bildet er den Boden der Auslaßkammer 13.

Die Kolbeneinheit aus Pumpkolben 8 mit Kolbenschaft 20 und gesondert aufgestecktem Verschlußkörper 22 ist ausschließlich durch den Steckeingriff des Kolbenschaftes 20 in den ihn im wesentlichen vollständig aufnehmenden Tragansatz 19 und die Axialsicherung durch den Verschlußkörper 22 gebildet, der in einer Richtung spielfrei am Inhalt des Medienspeichers 12 anliegt und gegen die andere Richtung durch vorgespannten Eingriff in die Innenumfangsfläche gesichert ist, wodurch ebenfalls eine mit einer vorbestimmten Kraft überwindbare Rastung gebildet ist. Der Zylinder 7 ist seinerseits ausschließlich durch den Eingriff in den Außenumfang des ringscheibenförmigen Kolbens 8 frei getragen. Außer der Pumpe 6 mit Tragschaft 19 ist der Grundkörper 2 im Innern von Einbauten, wie z.B. Versteifungsrippen oder dgl., völlig frei, so daß er, obwohl im wesentlichen formsteif, an seinem hinteren Ende quer zur Mittelachse 10 elastisch zusammendrückbar ist.

Am hinteren, auf voller Weite offenen Ende des Gehäuses 5 bildet der Grundkörper 2 eine zweigeteilte, ebene und zur Mittelachse 10 rechtwinklige Endfläche 33, wobei in Ansicht gemäß Fig. 1 der Zylinder 7 mit Abstand im wesentlichen zwischen den Teilflächen liegt. Die Außenseite des Bodens des Zylinders 7 bildet eine Finger-Druckfläche bzw. Handhabe 34, die in der Ausgangslage gegenüber der Ebene der Endfläche 33 geringfügig zurückversetzt ist. Dadurch kann die Austragvorrichtung 1 ungehindert mit der Endfläche 33 auf eine Standfläche gestellt werden.

Im Mantel des Gehäuses 5 sind zwei einander gegenüberliegende, von der Endfläche 33 ausgehende, über den größeren Teil der Gehäusehöhe sowie der Gehäusebreite reichende, gleiche, miteinander fluchtende, von der Endfläche 33 nach vorne in der Breite abnehmende, konkav begrenzte und gegenüber dem Durchmesser des Zylinders 7 wesentlich breitere Ausschnitte 36 für den Eingriff eines Daumens vorgesehen, deren Breite etwa 20 mm oder weniger betragen kann und deren Abstand voneinander demgegenüber wesentlich kleiner ist. Das Gehäuse 5 ist nämlich in Axialansicht flachlänglich mit zwei gegenüberliegenden, konvex gekrümmten, beiderseits einer Mittelebene 40 liegenden Flachwandungen 37, deren Krümmungsradius wesentlich größer als die Gehäusedicke quer zur Ebene 40 ist. Die beiden Flachwandungen 37 gehen über konvex und wesentlich stärker gekrümmte, an die Teilflächen der Endfläche 33 anschließende Seitenkanten 38 ineinander über, die beiderseits einer zur Ebene 40 rechtwinkligen Mittelebene 41 liegen, welche wie die Ebene 40 eine Axialebene der Mittelachse 10 bildet. Das Gehäuse 5 weist flachelliptische Querschnitte auf und steht in Axialansicht allseits über den Außenumfang des im wesentlichen kreisrunde Querschnitte aufweisenden Halses 3 vor.

Aufgrund der Masseverteilung und der Schwerpunktlage steht die Austragvorrichtung 1 auf der Endfläche 33 weniger kippstabil als sie auf einer der beiden Flachwandungen 37 liegt, wobei die drei sich dadurch ergebenden Lagen die einzigen stabilen Lagen sind. Die Erstreckung des Gehäuses 5 zwischen den Seitenkanten 38 ist zwei- bis dreimal größer als die Erstreckung quer dazu, wobei die Einzelflächen der Standfläche 33 annähernd U- bzw. V-förmig mit gegeneinander gerichteten Schenkeln sind.

Die einteilig an den Hals 3 anschließende Stirnwand des Gehäuses 5 bildet beiderseits des Halses 3 jeweils eine Handhabe 35 in Form einer Finger-Druckfläche, die jeweils durch einen Endabschnitt der länglichen und über den Außenumfang des Gehäusemantels nicht vorstehenden Stirnwand gebildet ist. Die in Richtung der Mittelachse 10 höchstens um die Gehäusehöhe im Abstand zueinanderliegenden Handhaben 34, 35 bzw. Druckflächen liegen quer zu den beiden, zur Mittelebene 40 parallelen Liegeebenen, so daß die Austragvorrichtung 1 mit drei Fingern leicht aus dem liegenden Zustand gegriffen werden kann. Die Handhaben 35 liegen im wesentlichen fluchtend mit den Teilflächen der Endfläche 33, in deren Bereich das Gehäuse 5 die größte Festigkeit hat.

Durch die beschriebene Ausbildung hat die Austragvorrichtung 1 in Ansicht im wesentlichen T-Form mit vom T-Schaft weg abgewinkelten Enden des T-Quersteges, wobei diese Enden beiderseits der Ausschnitte 36 liegende, frei abstehende, V-förmig profilierte Gehäuse-Finger bilden, auf deren Endflächen die Austragvorrichtung gestellt werden kann. Der Außendurchmesser des Zylinders 7 ist nur geringfügig kleiner als der lichte Abstand zwischen den Flachwandungen 37. Die beschriebenen Abschnitte des Gehäuses sind einzeln oder alle im wesentlichen formstabil bzw. rückfedernd elastisch biegbar.

Der Stutzen 4 kann mit einem kappenförmigen Deckel 42 verschlossen sein, der auf den abgesetzten Außenumfang des Stutzens 4 mit seinem Mantel so aufgesteckt ist, daß sein Außenumfang bündig in den Außenumfang des Halses 3 übergeht, so daß ein versehentliches Abstreifen vermieden ist.

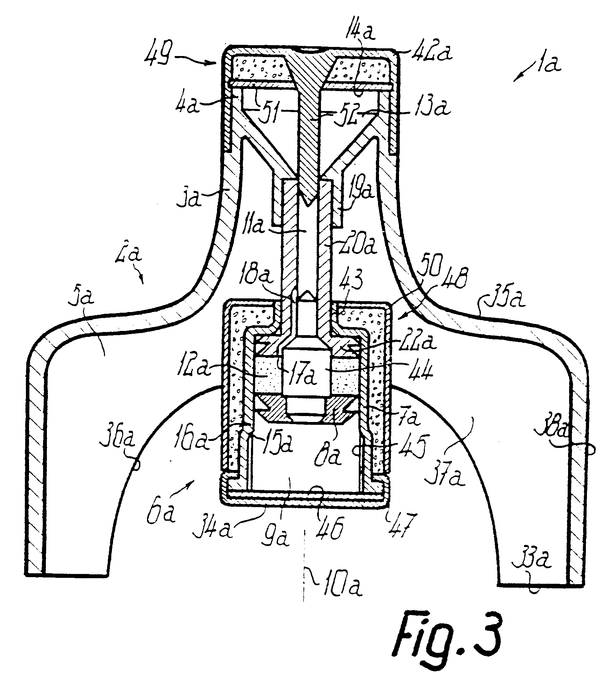

In Fig. 3 sind für entsprechende Teile die gleichen Bezugszeichen, jedoch mit dem Index "a" verwendet, weshalb alle Beschreibungsteile entsprechend gelten. Die Medienkammer 12a ist in diesem Fall innerhalb eines unmittelbar vor der Pumpkammer 9a liegenden Abschnittes des Zylindergehäuses 7a vorgesehen und von der Pumpkammer 9a lediglich durch den Pumpkolben 8a getrennt. Am anderen, vorderen Ende ist die Medienkammer 12a von einem Verschlußkolben 22a begrenzt, der an derselben Kolbeneinheit wie der Pumpkolben 8a vorgesehen und mit diesem axial bewegbar ist. Beide Kolben haben gleiche Außendurchmesser, und der Verschlußkolben 22a liegt in der Ausgangsstellung an einer vorderen Stirnwand des Zylindergehäuses 7a an. Diese Stirnwand wird im Bereich eines nach außen vorstehenden Führungs-Halses 43 von dem unmittelbar an den Verschlußkolben 22a anschließenden Kolbenschaft 20a durchsetzt.

In den Verschlußkolben 22a bzw. den Kolbenschaft 20a ist der Pumpkolben 8a mit einem Schaftteil 44 so eingesteckt, daß zwischen diesen und der einteilig aus Verschlußkolben 22a und Kolbenschaft 20a bestehenden Baueinheit mindestens ein Labyrinthkanal 18a gebildet ist. Dieser, am Außenumfang des Schaftteiles 44 liegende und durch eine mehrfach abgewinkelte Innennut gebildete Labyrinthkanal 18a reicht von dem in der hinteren Stirnfläche des Verschlußkolbens 22a liegenden Auslaß 17a bis in den auf voller Innenweite offenen Auslaßkanal 11a und ist hinsichtlich seiner Weite an die Fließfähigkeit des gespeicherten Mediums angepaßt. Der Pumpkolben 8a kann einteilig mit dem Schaftteil 44 oder getrennt ausgebildet sein, das mit einem im Durchmesser reduzierten Schaftansatz in den Auslaßkanal 11a eingreift.

Zur Bildung des Einlaßventiles 16a sind am Innenumfang der Zylinderlaufbahn Schlitze 45, insbesondere Längsschlitze, vorgesehen, zu deren den Einlaß 15a bildenden Enden der Pumpkolben 8a in der Ausgangslage im Abstand liegt. Wird der Pumpkolben 8a im Zylinder 7a verschoben, so wird zunächst die Luft in der Pumpkammer 9a verdichtet, bis alle Kolbenlippen des Pumpkolbens 8a den Einlaß 15a überlaufen und dadurch nach Art eines Schiebeventiles zur mitlaufenden Medienkammer 12a geöffnet haben. Das Zylindergehäuse 7a kann einen zum hinteren, seinem Boden zugehörigen Ende offenen Zylinderkörper aufweisen, der an diesem Ende unter Zwischenlage einer Dichtscheibe 46 mit einem z.B. auf einen Endflansch aufgekrimmten Deckel 47 verschlossen ist. Dadurch kann der Verschlußkolben 22a von diesem Ende her, vorzugsweise gemeinsam mit dem Schaftteil 44 eingesetzt werden, wonach das zu speichernde Medium eingefüllt, dann die Medienkammer 12a durch Einsetzen des Pumpkolbens 8a verschlossen und schließlich der Deckel 47 angebracht und dadurch die Pumpkammer 9a dicht verschlossen wird.

Für mindestens ein gespeichertes Medium der Austragvorrichtung 1a kann eine Einrichtung 48 bzw. 49 zur physikalischen bzw. chemischen Beeinflussung, insbesondere zur Trockenhaltung, vorgesehen sein. Die im wesentlichen den Zylinder 7a umgebende und an diesem angeordnete Einrichtung 48 weist ein auf das Zylindergehäuse 7a aufgestecktes, dünnwandiges Außengehäuse 50 auf, das an einer Stirnwand durch den Hals 43 und am anderen Ende durch Anlage am Deckel 47 verschlossen ist.

In diesem Gehäuse 50 ist ein Trocknungsmittel vorgesehen, das sowohl auf den Inhalt der Medienkammer 12a als auch auf denjenigen der Pumpkammer 9a wirkt. Ein entsprechendes Gehäuse kann z.B. auch im abnehmbaren Deckel 42a vorgesehen sein und das Innere der Auslaßkammer 13a trocken halten. Dieses Gehäuse der Einrichtung 49 ist vom Mantel und der Stirnwand des Deckels 42a sowie einer dieser gegenüberliegend in den Mantel eingesprengten Stirnwand 51 begrenzt, mit welcher der Deckel 42a an der Stirnfläche des Stutzens 4 anliegt. Die Pumpe 6a ist in diesem Fall lediglich durch Einstecken des Kolbenschaftes 20a in dem verhältnismäßig kurzen, vollständig innerhalb des Halses 3a liegenden Tragansatz 19a befestigt. Der Deckel kann einen über seine Innenseite vorstehenden Verschlußdorn 52 aufweisen, der am zugehörigen Ende des Kolbenschaftes 20a in den Auslaßkanal 11a eingesteckt ist und beim Abnehmen des Deckels 42a selbsttätig herausgezogen wird.

## Patentansprüche

1. Austragvorrichtung (1, 1a) für Medien, mit einem Grundkörper (2, 2a), mindestens einer handbetätigbaren und einen Betätigungsschaft (19, 19a) einschließenden Pumpe (6, 6a), Wenigstens einer Pumpkammer (9, 9a), mindestens einem zu einer Austragöffnung (14, 14a) führenden Auslaßkanal (11, 11a, 18a), wenigstens einem Medienraum (12, 12a) für das flüssige und/oder pulverförmige Medium, einer gegenüber dem Medienraum (12, 12a) manuell verschiebbaren Kolbeneinheit mit wenigstens einem kolbenartigen, mit dem Betätigungsschaft (19, 19a) zu bewegenden Bauteil (8, 22, 8a, 22a), und mit einem einen Verschluß des Medienraumes (12, 12a) und eine Begrenzung des Auslaßkanales (11, 16, 18a) bildenden Füllstück (21, 44), wobei mindestens ein kolbenartiger Bauteil (8, 22, 8a, 22a) durch Stecken an dem Füllstück (21, 44) angeordnet ist, dadurch gekennzeichnet, daß der Betätigungsschaft (19, 19a) zur Betätigung der Pumpe (6, 6a) vorgesehen ist und daß der Betätigungsschaft (19, 19a) den Auslaßkanal (11, 11a, 18a) umgibt.

2. Austragvorrichtung nach Anspruch 1, dadurch gekennzeichnet, daß der Medienraum (12, 12a) in mindestens einer Stellung der Austragvorrichtung (1, 1a) einen im Querschnitt erweiterten Abschnitt des von der Pumpkammer (9, 9a) zur Austragöffnung (14, 14a) führenden Auslaßkanales (11, 11a) bildet, daß insbesondere wenigstens ein Medienraum (12, 12a) von der jeweiligen Pumpkammer (9, 9a) gesondert vorgesehen ist, daß vorzugsweise mindestens ein Medienraum (12, 12a) in Wenigstens einer Stellung der Austragvorrichtung (1, 1a) mit der Austragöffnung (14, 14a) und/oder der Pumpkammer (9, 9a) leitungsverbunden bzw. in Wenigstens einer weiteren Stellung der Austragvorrichtung (1, 1a) im wesentlichen vollständig dicht geschlossen ist und daß insbesondere das Füllstück (21, 44) den Medienraum (12, 12a) durchsetzt bzw. in dem Medienraum (12, 12a) bewegbar angeordnet ist.

3. Austragvorrichtung nach Anspruch 1 oder 2, dadurch gekennzeichnet, daß der Medienraum (12, 12a) im wesentlichen achsgleich zur Pumpe (6, 6a), zum Auslaßkanal (11, 11a) oder zur Auslaßöffnung (14, 14a), näher bei der Austragöffnung (14, 14a) als die Pumpe (6, 6a), innerhalb mindestens eines sie im Abstand umgebenden Gehäuses (3, 5a), im Anschluß an mindestens ein Ventil (16, 18; 16a) bzw. einen Labyrinthverschluß (18a), in direkter Leitungsverbindung mit der Pumpe (6, 6a), unmittelbar benachbart zur Austragöffnung (14) bzw. zur Pumpe (6a), im Anschluß an mindestens einen spaltartigen Abschnitt eines Kanales (11, 11a) und/oder achsgleich zu einer Mittelachse (10, 10a) der Austragvorrichtung liegt, und daß insbesondere ein in den Auslaßkanal (11a) reichender Labyrinthkanal (18a) zwischen dem Füllstück (44) und einem Kolben (22a) am Außenumfang des Füllstückes (44) gebildet und/oder in den Auslaßkanal (18a) ein Verschlußdorn (52) eingesteckt ist.

4. Austragvorrichtung nach einem der vorherigen Ansprüche, dadurch gekennzeichnet, daß ein Auslaß (17) und/oder ein Einlaß (15, 15a) des Medienraumes (12, 12a) mit einem Ventil (18, 16 bzw. 16a), insbesondere einem mit der Betätigungshandhabe für die Pumpe (6, 6a) betätigbaren Schieberventil, gesteuert ist, daß vorzugsweise simultan betätigbare Ventile (16, 18) im Bereich gegenüberliegender Enden des Medienraumes (12) vorgesehen sind, und daß insbesondere mindestens ein Ventilkörper (22) durch einen etwa der lichten Weite des Medienraumes (12) entsprechenden, kolbenartigen Stirndeckel gebildet ist und/oder daß ein Ventilkörper (22) an dem Füllstück (21) vorgesehen bzw. durch das Füllstück (21) gebildet ist, daß insbesondere ein Ventilkörper (22) als Ringkörper ausgebildet ist und daß vorzugsweise ein in der Kolbenstirnfläche eines kolbenartigen Bauteiles (8) mündender Kanalabschnitt (23) an das hintere Ende eines von einem Schaftabschnitt des Füllstückes (21) und einem Tragansatz (19) für einen kolbenartigen Bauteil (8) begrenzten Kanalabschnittes (25) anschließt.

5. Austragvorrichtung nach einem der vorherigen Ansprüche, dadurch gekennzeichnet, daß der Medienraum (12a) am Außenumfang im wesentlichen von einem Zylindergehäuse (7a) der Pumpe (6a) begrenzt ist, daß insbesondere der Medienraum (12a) von der Pumpkammer (9a) im wesentlichen ausschließlich durch einen Pumpkolben (8a) getrennt ist, und daß vorzugsweise der Pumpkolben (8a) mit Übertrittsschlitzen (45) in der Zylinderwandung als Ventilkörper ein Einlaßventil (16a) bildet und/oder daß an das hintere Ende des Medienraumes (12) ein Rohransatz anschließt, daß insbesondere in einem frei vorstehenden, die Austragöffnung (14) und einen Stutzen (4) bildenden Hals (3) des Grundkörpers (2) eine Schubkolben-Pumpe (6) angeordnet ist, und daß vorzugsweise der Medienraum (12) benachbart zum hinteren Ende des Stutzens (4) liegt.

6. Austragvorrichtung nach einem der vorherigen Ansprüche, dadurch gekennzeichnet, daß eine, insbesondere mit einem Pumphub, verschiebbare Lagerung des Medienraumes (12a) vorgesehen ist, daß vorzugsweise der Medienraum (12a) an gegenüberliegenden Enden von entlang eines Kammermantels bzw. eines Pumpenzylinders verschiebbaren Kolben (8a, 22a) begrenzt ist und daß insbesondere eine Folgeschaltung (26) der Betätigung der Pumpe (6, 6a) und der Öffnung des Medienraumes (12, 12a) bzw. vor Öffnung der Pumpkammer (9) zum Medienraum (12) eine Vorkompressionsstufe für die Pumpkammer (9) vorgesehen ist, und/oder daß eine Pumpe (6) ein einteilig napfförmiges bzw. zum hinteren Boden-Ende offenes und ggf. nach Einfüllen des Mediums bzw. Einsetzen eines kolbenartigen Bauteiles (22a) verschlossenes Zylindergehäuse (7 bzw. 7a) aufweist, daß insbesondere ein Zylindergehäuse (7, 7a) bzw. ein Pumpkolben (8) über eine reine Steckverbindung an dem Grundkörper (2, 2a) befestigt ist und daß vorzugsweise der Grundkörper (2, 2a) ein das Zylindergehäuse (7, 7a) aufnehmendes Gehäuse (5, 5a) mit einer hinteren Endfläche (33, 33a) bildet, gegenüber welcher insbesondere die Außenfläche (34, 34a) eines Bodens des Zylindergehäuses (7, 7a) in der Ausgangslage zurückversetzt ist.

7. Austragvorrichtung nach einem der vorherigen Ansprüche, dadurch gekennzeichnet, daß die Pumpe (6, 6a) in einer Ausgangsstellung, insbesondere federnd gerastet, zur Einleitung des Pumphubes überwindbar gegen Betätigung arretiert ist und daß vorzugsweise durch Betätigung der Pumpe (6, 6a) ein Öffnen des Medienraumes (12, 12a) vorgesehen, wie wenigstens ein Ventil (16, 18) für den Medienraum (12) betätigbar bzw. etwa bei Beginn einer Pumpmitnahme geöffnet ist, daß insbesondere das Füllstück (21) anschlagbegrenzt an einem Gehäusehals (19) eines Grundkörpers (2) gelagert bzw. ein Kolben (8) gegenüber einem Rastglied (30) einer Zylinderwandung arretiert ist, und/oder daß zur vorangehenden Öffnung eines Auslasses (17) des Medienraumes (12) und zum sofort nachfolgenden Beginn des Pumpbetriebes eine vordere Endfläche eines Tragansatzes (19) für einen Pumpkolben (8) eine einer Anschlagfläche (28) des Pumpkolbens (8) um einen Ventil-Öffnungsweg eines Auslaßventiles (18) gegenüberliegende Mitnahmefläche (27) bildet, daß insbesondere der Pumpkolben (8) zur Mitnahme über den Öffnungsweg über eine Hemmung in einen Innenumfang eines Zylindergehäuses (7) eingreift und daß vorzugsweise eine Lagesicherung mindestens eines kolbenartigen Bauteiles (8, 22) ineinandergreifende Rastglieder (30, 31), wie einen Rastnocken (30) am Zylindergehäuse (7) und eine Rastnut (31), aufweist.

8. Austragvorrichtung nach einem der vorherigen Ansprüche, dadurch gekennzeichnet, daß der Medienraum (12, 12a) und/oder ein Einlaß (15, 15a) in die Medienkammer (12, 12a) eine im Querschnitt kranz- bzw. ringförmige Anordnung aufweisen, insbesondere koaxial sind, daß vorzugsweise das Zentrum des Medienraumes (12, 12a) bzw. des Einlasses (15, 15a) von einem Schaft, wie einem Kolbenschaft (20), durchsetzt ist, und/oder daß das Medium beim Austrag von dem Medienraum (12, 12a) durch einen Auslaß (17a) in einen Auslaßkanal (11a) und dann durch die Auslaßöffnung (14, 14a) gelangt, daß insbesondere der Auslaßkanal (11) ringförmig begrenzt ist und daß vorzugsweise das Medium beim Austrag von einem Ende des Medienraumes (12, 12a) zum gegenüberliegenden Ende herausgedrückt wird.

9. Austragvorrichtung nach einem der vorherigen Ansprüche, dadurch gekennzeichnet, daß wenigstens dem Auslaß (17, 17a) des Medienraumes (12, 12a) bzw. der Austragöffnung (14, 14a) eine Verwirbelungseinrichtung für das Medium zugeordnet ist, daß insbesondere in einer die Auslaßöffnung (14, 14a) bildenden Verwirbelungs- oder Austragkammer (13) ein Prallteller (22) oder dgl. vorgesehen ist, und daß vorzugsweise der Prallteller (22) oder dgl. aus dem Medienraum (12) herausschiebbar bzw. durch einen Verschlußdeckel für den Medienraum (12) gebildet ist, und/oder daß unmittelbar an den Auslaß (17a) ein Labyrinthkanal (18a) angeschlossen ist, daß insbesondere der Labyrinthkanal (18a) von einer hinteren Stirnfläche eines den Medienraum (12a) verschließenden, kolbenartigen Bauteiles (22a) ausgeht und daß vorzugsweise der Labyrinthkanal (18a) zum Durchlaß des Mediums nur unter Betriebsdruck hinsichtlich seiner Weite an die Fließfähigkeit des Mediums angepaßt ist.

10. Austragvorrichtung nach einem der vorherigen Ansprüche, dadurch gekennzeichnet, daß der Medienraum (12, 12a) als Speicher für das pulverförmige bzw. flüssige Medium vorgesehen ist, daß insbesondere der Medienraum (12, 12a) und die Pumpkammer (9, 9a) für unterschiedliche Medien vorgesehen sind bzw. die Pumpkammer (9, 9a) als einlaßfreie, dem Pumpkolben (8, 8a) gegenüberliegend geschlossene und eine Betätigungshandhabe (34, 34a) bildende Pumpkammer vorgesehen ist, und daß vorzugsweise die Auslaßöffnung (14, 14a) eine größere lichte Weite als der Auslaßkanal (11, 11a), der Medienraum (12, 12a) und/oder die Pumpkammer (9a) aufweist.

11. Austragvorrichtung nach einem der vorherigen Ansprüche, dadurch gekennzeichnet, daß mindestens eine Einrichtung (48, 49) zur Trockenhaltung oder dgl. mindestens eines gespeicherten Mediums, von medienführenden Flächen der Vorrichtung oder dgl. vorgesehen ist, wobei vorzugsweise mindestens eine mit einem Trocknungsmittel gefüllte Kammer vorgesehen ist.

12. Austragvorrichtung nach einem der vorherigen Ansprüche, dadurch gekennzeichnet, daß die Pumpe (6, 6a) bzw. der Medienraum (12, 12a) in Wenigstens einer Stellung, insbesondere bereits in der Ausgangsstellung, im wesentlichen vollständig innerhalb eines Gehäuses (5, 5a) des Grundkörpers (2, 2a) liegt, in das sie etwa in Längsrichtung eines Austragstutzens (4, 4a) hineinragt, und daß vorzugsweise die Pumpe (6, 6a) bzw. der Medienraum (12, 12a) als Baueinheit über mindestens eine Steckverbindung miteinander bzw. mit dem insbesondere ein Außengehäuse (5, 5a) bildenden Grundkörper (2, 2a) verbunden sind.

13. Austragvorrichtung nach einem der vorherigen Ansprüche, dadurch gekennzeichnet, daß ein Schaft, wie ein einziger Kolbenschaft, (20, 20a), der Pumpe (6, 6a) in einen den Betätigungsschaft bildenden Tragansatz (19, 19a) des Grundkörpers (2, 2a) eingesetzt ist, daß insbesondere der rohrförmige, den Medienraum (12) umgebende, Tragansatz (19, 19a) im Wesentlichen berührungsfrei innerhalb eines Halses (3, 3a) bzw. teilweise in einem Gehäuse (5, 5a) des Grundkörpers (2, 2a) liegt, und daß vorzugsweise der Tragansatz (19, 19a) mit seinem vorderen Ende einteilig in den mit seinem Ende einen Austragstutzen (4, 4a) bildenden, frei vorstehenden Hals (3, 3a) übergeht, und/oder daß in einen Tragansatz (19, 19a) des Grundkörpers (2, 2a) ein das Füllstück (21, 44) aufweisender Kolbenschaft (20, 20a) eingesteckt ist, daß insbesondere ein Kolbenschaft (20, 20a) abgedichtet in den Tragansatz (19, 19a) eingesetzt ist und daß vorzugsweise am Ende des Pumphubes ein Pumpkolben (8, 8a) am Boden der Pumpenkammer (9, 9a) anliegt bzw. das Zylindergehäuse (7, 7a) zum größten Teil innerhalb des Halses (3, 3a) liegt.

14. Austragvorrichtung nach einem der vorherigen Ansprüche, dadurch gekennzeichnet, daß ein Pumpkolben (8) mit einem bewegbaren Ventilkörper (22) eines Auslaßventiles (18) des Medienraumes (12) eine Baueinheit bildet, daß insbesondere die Baueinheit ausschließlich durch Steckeingriff an einem Tragansatz (19) des Grundkörpers (2) angeordnet ist, und daß vorzugsweise eine Axialsicherung der Baueinheit durch spielfreie Anlage des Ventilkörpers (22) am Medieninhalt des Medienraumes (12) gebildet ist und/oder daß ein den Medienraum (12a) bildendes Zylindergehäuse (7a) annähernd ausschließlich durch Eingriff in den Außenumfang eines ringförmigen Pumpkolbens (8a) frei getragen ist, daß insbesondere eine Kolbeneinheit aus Pumpkolben (8) und Kolbenschaft (20) einen gesondert aufgesteckten Ventilkörper (22) aufweist und daß vorzugsweise der Kolbenschaft (20) durch Steckeingriff im wesentlichen vollständig in einem Tragansatz (19) des Grundkörpers (2) aufgenommen ist.

15. Austragvorrichtung nach einem der vorherigen Ansprüche, dadurch gekennzeichnet, daß ein Gehäuse (5, 5a) der Austragvorrichtung (1, 1a) - in Richtung einer Mittelachse (10, 10a) gesehen - länglich gestreckt und insbesondere mit zwei gegenüberliegenden flacheren Seiten (37) versehen ist, daß vorzugsweise mindestens eine der flacheren Seiten (37) zur Bildung eines Fingereingriffes von einem Eingriffs-Ausschnitt (36) durchsetzt ist, und daß insbesondere außer den flacheren Seiten (37) eine weitere Standfläche (33) zur gegenüber den flacheren Seiten (37) weniger lagestabilen Anordnung der Austragvorrichtung (1, 1a) auf einer durchgehenden Stützfläche vorgesehen bzw. die weitere Standfläche (33) durch eine hintere Endfläche des Grundkörpers (2, 2a) gebildet ist, und/oder daß der Grundkörper (2, 2a) eine zweigeteilte hintere Endfläche (33, 33a) bildet, daß insbesondere in Seitenansicht ein den Medienraum (12a) bildendes Zylindergehäuse (7a) mit Abstand im wesentlichen zwischen den Teilflächen der Endfläche (33a) liegt und daß vorzugsweise der Abstand zwischen zwei einander gegenüberliegenden Ausschnitten (36) in einem Mantel des Grundkörpers (2) kleiner als ihre Breite bzw. diese Breite gegenüber dem Durchmesser des Zylindergehäuses (7a) größer ist.

16. Austragvorrichtung nach einem der vorherigen Ansprüche, dadurch gekennzeichnet, daß zwei Flachwandungen (37) des Mantels eines Gehäuses (5) des Grundkörpers (2) über konvex stark gekrümmte Seitenkanten (38) ineinander übergehen, daß insbesondere in Axialansicht das Gehäuse (5) allseits über den Außenumfang eines einen Austragstutzen (4) bildenden Halses (3) des Grundkörpers (2) vorsteht und daß vorzugsweise die Seitenkanten (38) an Teilflächen einer hinteren Endfläche (33) des Gehäuses (5) anschließen, Welche annähernd U- bzw. V-förmig mit gegeneinander gerichteten Schenkeln sind, und/oder daß die Teilfächen (33) im wesentlichen fluchtend mit Betätigungs-Handhaben (35) des Grundkörpers (2) liegen, daß insbesondere die Austragvorrichtung (1) in Seitenansicht im wesentlichen T- bzw. Y-förmig ist, und daß vorzugsweise der T-Quersteg der T-Form vom T-Schaft weg abgewinkelte sowie frei abstehende Enden bildet.

17. Austragvorrichtung nach einem der vorherigen Ansprüche, dadurch gekennzeichnet, daß voneinander weggerichtete, Finger-Druckflächen (34, 35 bzw. 34a, 35a) bildende, Betätigungs-Handhaben vorgesehen sind, und daß vorzugsweise zwei Betätigungs-Handhaben mit gleicher Druckrichtung beiderseits eines Austragstutzens (4, 4a) durch eine Stirnwand eines Außengehäuses (5, 5a) bzw. eine Betätigungs-Handhabe (34) durch einen im wesentlichen innerhalb des Gehäuses (5, 5a) liegenden Schiebekörper (7) gebildet sind, und/oder daß die Austragvorrichtung zum Tragen sowie gleichzeitigen Betätigen mit einer einzigen Hand ausgebildet ist, daß insbesondere zwei Handhaben (35, 35a) durch Endabschnitte einer länglichen Stirnwand des Grundkörpers (2, 2a) gebildet sind und daß vorzugsweise die Austragvorrichtung (1, 1a) in Axialansicht länglich gestreckt ist.

18. Austragvorrichtung nach einem der vorherigen Ansprüche, dadurch gekennzeichnet, daß für die Austragöffnung (14, 14a) ein abnehmbarer, insbesondere kappenförmiger Deckel (42, 42a) vorgesehen ist, und daß vorzugsweise der Deckel (42, 42a) über seine Innenseite vorstehend ein Verschlußglied (52), wie einen Dorn an einer Stirnwand für den unmittelbaren Eingriff in einen gegenüber einer Austragkammer (13a) engeren Einlaß, aufweist, und/oder daß ein Deckel (42, 42a) auf den Außenumfang eines Stutzens (4) des Grundkörpers (2) aufgesteckt ist, daß insbesondere der Außenumfang abgesetzt ist und daß vorzugsweise der Deckel (42, 42a) an einer Stirnfläche anliegt bzw. zur Sicherung gegen versehentliches Abstreifen in einen den Stutzen (4) bildenden Hals (3) übergeht.

19. Austragvorrichtung nach einem der vorherigen Ansprüche, dadurch gekennzeichnet, daß sie als Einweg- und/oder Einmal-Austragvorrichtung (1, 1a) zum Austrag einer festen Speichermenge eines pulverförmigen bzw. flüssigen Mediums in Teilstrecken bzw. einem Durchgang eines einzigen Gesamt-Pumphubes, insbesondere mit einem zusätzlichen Fördermedium, ausgebildet ist und daß die Speichermenge vorzugsweise ausschließlich durch Öffnung eines Medienraumes (12, 12a) freigegeben wird.

## Claims

1. Dispenser (1, 1a) for media, with a body (2, 2a), at least one manually operable pump (6, 6a) enclosing an operating shaft (19, 19a), at least one pump chamber (9, 9a), at least one outlet channel (11, 11a, 18a) leading to a dispensing opening (14, 14a), at least one medium chamber (12, 12a) for the liquid and/or pulverulent medium, a piston unit manually closable with respect to the medium chamber (12, 12a) and having at least one piston-like component (8, 22, 8a, 22a) to be moved together with the operating shaft (19, 19a), and with a filling piece (21, 44) forming a closure of the medium chamber (12, 12a) and a limitation of the outlet channel (11, 16, 18a), at least one piston-like component (8, 22, 8a, 22a) being positioned by fitting on the filling piece (21, 44), characterized in that the operating shaft (19, 19a) is provided for operating the pump (6, 6a) and that the operating shaft (19, 19a) surrounds the outlet channel (11, 11a, 18a).

2. Dispenser according to claim 1, characterized in that medium chamber (12, 12a) in at least one position of the dispenser (1, 1a), forms a cross-sectionally widened portion of the outlet channel (11, 11a) leading from the pump chamber (9, 9a) to the dispensing opening (14, 14a), that in particular at least one medium chamber (12, 12a) is provided separate from the pump chamber (9, 9a), that preferably at least one medium chamber (12, 12a) in at least one position of the dispenser (1, 1a) is line-connected with the dispensing opening (14, 14a) and/or the pump chamber (9, 9a) and in at least one further position of the dispenser (1, 1a) is substantially completely tightly closed and that in particular the filling piece (21, 44) traverses the medium chamber (12, 12a) or is movably placed in the medium chamber (12, 12a).

3. Dispenser according to claim 1 or 2, characterized in that the medium chamber (12, 12a) is substantially equiaxial to pump (6, 6a), to the outlet channel (11, 11a) or the outlet opening (14, 14a), closer to the dispensing opening (14, 14a) than the pump (6, 6a), within at least one casing (3, 5a) spacedly surrounding the same, follows onto at least one valve (16, 18; 16a) or a labyrinth closure (18a), is in direct line connection to the pump (6, 6a) immediately adjacent to the dispensing opening (14) or to the pump (6a), connects onto at least one gap-like portion of a channel (11, 11a) and/or is equiaxial to a median axis (10, 10a) of the dispenser and that in particular a labyrinth channel (18a) extending into the outlet channel (11a) between the filling piece (44) and a piston (22a) is formed on the outer circumference of the filling piece (44) and/or a closure mandrel (52) is inserted in the outlet channel (18a).

4. Dispenser according to one of the preceding claims, characterized in that an outlet (17) and/or an inlet (15, 15a) of the medium chamber (12, 12a) is controlled with a valve (18, 16 or 16a), particularly with a slide valve operable with the operating handle for the pump (6, 6a), that preferably simultaneously operable valves (16, 18) are provided in the vicinity of opposite ends of the medium chamber (12) and that in particular at least one valve body (22) is formed by a piston-like end cover corresponding roughly to the inside diameter of the medium chamber (12) and/or that a valve body (22) is provided on the filling piece (21) or is formed by the filling piece (21), that in particular a valve body (22) is constructed as an annular body and that preferably a channel portion (23) issuing into the piston end face of a piston-like component (8) is connected to the rear end of a channel portion (25) bounded by a shaft portion of the filling piece (21) and a support attachment (19) for a piston-like component (8).

5. Dispenser according to one of the preceding claims, characterized in that the medium chamber (12a) is bounded on the outer circumference substantially by a cylinder casing (7a) of the pump (6a), that in particular the medium chamber (12a) is separated from the pump chamber (9a) substantially exclusively by a pump piston (8a) and that preferably the pump piston (8a) with passage slots (45) in the cylinder wall forms as the valve body an inlet valve (16a) and/or on the rear end of the medium chamber (12) is connected a tubular attachment, that in particular in a freely projecting neck (3) of the body (2) forming the dispensing opening (14) and a connecting piece (4) is located a thrust piston pump (6) and that preferably the medium chamber (12) is adjacent to the rear end of the connecting piece (4).

6. Dispenser according to one of the preceding claims, characterized in that a mounting of the medium chamber (12a), more particularly displaceable with a pump stroke is provided and is bounded on opposite ends by pistons (8a, 22a) displaceable along a chamber jacket or a pump cylinder and that in particular a sequence control (26) is provided for operating the pump (6, 6a) and the opening of the medium chamber (12, 12a) or prior to the opening of the pump chamber (9) to the medium chamber (12) a precompression stage is provided for the pump chamber (9) and/or that a pump (6) has a one-piece, cup-shaped cylinder casing (7 or 7a) or which is open towards the rear bottom end and closed optionally after filling the medium or inserting a piston-like component (22a), that in particular a cylinder casing (7, 7a) or a pump piston (8 ) is fixed by means of a pure plug connection to a body (2, 2a) and that preferably the body (2, 2a) forms a casing (5, 5a) with a rear end face (33, 33a) receiving the cylinder casing (7, 7a) and with respect to which is set back into the starting position the outer face (34, 34a) of a base of the cylinder casing (7, 7a).

7. Dispenser according to one of the preceding claims, characterized in that the pump (6, 6a) in a starting position is in particular resiliently locked against operation which can be overcome for initiating the pump stroke and that preferably by operating the pump (6, 6a) there is an opening of the medium chamber (12, 12a) and at least one valve (16, 18) for the medium chamber (12) is operable or opened roughly at the start of a pump driving, that in particular the filling piece (21) is mounted in stop-limited manner on a casing neck (19) of a body (2) or a piston (8) is locked with respect to a locking member (30) of a cylinder wall and/or that for the preceding opening of an outlet (17) of the medium chamber (12) and for the immediately following start of the pump operation a front end face of a support attachment (19) for a pump piston (8) forms a driving face (27) facing a stop face (28) of the pump piston (8) about a valve opening path of an outlet valve (18), that in particular the pump piston (8) for driving purposes engages over the opening path via an inhibiting means in an inner circumference of a cylinder casing (7) and that preferably there is a position securing of at least one piston-like component (8, 22) has interengaging locking members (30, 31), such as a locking cam (30) on the cylinder casing (7) and a locking groove (31).

8. Dispenser according to one of the preceding claims, characterized in that the medium chamber (12, 12a) and/or an inlet (15, 15a) have in the medium chamber (12, 12a) a cross-sectionally ring-like or annular arrangement and are in particular coaxial, that preferably the centre of the medium chamber (12, 12a) or the inlet (15, 15a) is traversed by a shaft, such as a piston shaft (20), and/or that medium on discharge from the medium chamber (12, 12a) passes through an outlet (17a) into an outlet channel (11a) and then through the outlet opening (14, 14a), that in particular the outlet channel (11) is bounded in annular manner and that preferably the medium on discharge from one end of the medium chamber (12, 12a) is forced out to the opposite end.

9. Dispenser according to one of the preceding claims, characterized in that with at least the outlet (17, 17a) of the medium chamber (12, 12a) or the dispensing opening (14, 14a) is associated a turbulence-inducing mechanism for the medium, that in particular in a turbulence-inducing or discharge chamber (13) forming the outlet opening (14, 14a) is provided a baffle plate (22) or the like and that preferably the baffle plate (22) or the like can be moved out of the medium chamber (12) or is formed by a closure cover for the medium chamber (12) and/or that a labyrinth channel (18a) is connected directly to the outlet (17a), that in particular the labyrinth channel (18a) emanates from a rear end face of a piston-like component (22a) closing the medium chamber (12a) and that preferably the width of the labyrinth channel (18a) is only adapted to the medium flowability for the passage of said medium under operating pressure.

10. Dispenser according to one of the preceding claims, characterized in that the medium chamber (12, 12a) constitutes a reservoir for the pulverulent or liquid medium, that in particular the medium chamber (12, 12a) and the pump chamber (9, 9a) are provided for different media or the pump chamber (9, 9a) is provided as an inlet-free pump chamber facing the pump piston (8, 8a) and forming an operating handle (34, 34a) and that preferably the outlet opening (14, 14a) has a larger inside diameter than the outlet channel (11, 11a), the medium chamber (12, 12a) and/or the pump chamber (9a).

11. Dispenser according to one of the preceding claims, characterized in that there is at least one device (48, 49) for keeping dry or the like at least one stored medium, medium-carrying surfaces of the apparatus or the like and preferably there is at least one chamber filled with a drying agent.

12. Dispenser according to one of the preceding claims, characterized in that the pump (6, 6a) or the medium chamber (12, 12a) in at least one position, particularly in the starting position, is substantially completely located within a casing (5, 5a) of the body (2, 2a), into which it projects roughly in the longitudinal direction of a dispensing connecting piece (4, 4a) and that preferably the pump (6, 6a) or medium chamber (12, 12a) are interconnected by means of at least one plug connection or in particular with the body (2, 2a) forming an outer casing (5, 5a) so as to constitute a standard component.

13. Dispenser according to one of the preceding claims, characterized in that a shaft, such as a single piston shaft (20, 20a), of the pump (6, 6a) is inserted in a support attachment (19, 19a) of the body (2, 2a) forming the operating shaft, that in particular the tubular support attachment (19, 19a) surrounding the medium chamber (12) is located in substantially contact-free manner within a neck (3, 3a) or partly in a casing (5, 5a) of the body (2, 2a) and that preferably the support attachment (19, 19a) with its front end passes in one piece into the freely projecting neck (3, 3a) forming with its end a dispensing connecting piece (4, 4a) and/or that in a support attachment (19, 19a) of the body (2, 2a) is inserted a piston shaft (20, 20a) having the filling piece (21, 44), that in particular a piston shaft (20, 20a) is inserted in sealed manner in the support attachment (19, 19a) and that preferably at the end of the pump stroke a pump piston (8, 8a) engages on the bottom of the pump chamber (9, 9a) or the cylinder casing (7, 7a) is largely located within the neck (3, 3a).

14. Dispenser according to one of the preceding claims, characterized in that a pump piston (8) with a movable valve body (22) of an outlet valve (18) of the medium chamber (12) forms a standard component, that in particular the standard component is placed exclusively by plug engagement on a support attachment (19) of the body (2) and that preferably an axial securing of the standard component is provided by clearance-free engagement of the valve body (22) on the medium content of the medium chamber (12) and/or that a cylinder casing (7a) forming the medium chamber (12a) is freely supported approximately exclusively by engaging in the outer circumference of an annular pump piston (8a), that in particular piston unit formed by the pump piston (8) and piston shaft (20) has a separately fitted valve body (22) and that preferably the piston shaft (20) is received by plug engagement substantially completely in a support attachment (19) of the body (2).

15. Dispenser according to one of the preceding claims, characterized in that a casing (5, 5a) of the dispenser (1, 1a), considered in the direction of a median axis (10, 10a), is elongated and is in particular provided with two facing, flat sides (37), that preferably at least one of the flat sides (37) is traversed by an engagement cutout (36) for forming a finger engagement and that in particular apart from the flat sides (37) there is a further standing surface (33) for an arrangement of the dispenser (1, 1a) less positionally stable than the flat sides (37) on a through support surface or the further standing surface (33) is formed by a rear end face of the body (2, 2a) and/or that the body (2, 2a) forms a two-part, rear end face (33, 33a), that in particular in side view a cylinder casing (7a) forming the medium chamber (12a) is spaced substantially between the partial faces of the end face (33a) and that preferably the spacing between two facing cutouts (36) in a jacket of the body (2) is smaller than the width thereof or said width is greater than the diameter of the cylinder casing (7a).

16. Dispenser according to one of the preceding claims, characterized in that two flat walls (37) of the jacket of a casing (5) of the body (2) pass into one another via strongly convexly curved lateral edges (38), that in particular in axial view of the casing (5) a neck (3) of the body (2) forming a dispensing connecting piece (4) projects on all sides over the outer circumference and that preferably the lateral edges (38) are connected to partial faces of a rear end face (33) of the casing (5), which are approximately U or V-shaped with oppositely directed legs and/or that the partial faces (33) are substantially aligned with operating handles (35) of the body (2), that in particular the dispenser (1) in side view is substantially T or Y-shaped and that preferably the T cross web of the T shape has freely projecting ends angled away from the T-shaft.

17. Dispenser according to one of the preceding claims, characterized in that operating handles forming finger pressure faces (34, 35, 34a, 35a) directed against one another are provided and that preferably two operating handles with the same pressure direction are formed on either side of a dispensing connecting piece (4, 4a) by an end wall of an outer casing (5, 5a) or an operating handle (34) is formed by a sliding body (7) located substantially within the casing (5, 5a) and/or that the dispenser is constructed for carrying and simultaneously operating with one hand, that in particular there are two handles (35, 35a) formed by end portions of an elongated end wall of the body (2, 2a) and that preferably the dispenser (1, 1a) is elongated in axial view.

18. Dispenser according to one of the preceding claims, characterized in that for the outlet opening (14, 14a) is provided a removable, particularly a caplike lid (42, 42a) and that preferably the lid (42, 42a) has projecting over its inside a closure member (52), such as a mandrel on an end wall for direct engagement in an inlet narrower than a discharge chamber (13a) and/or that a lid (42, 42a) is mounted on the outer circumference of a connecting piece (4) of the body (2), that in particular the outer circumference is offset and preferably the lid (42, 42a) engages on an end face or passes into a neck (3) forming the connecting piece (4) for preventing accidental removal.

19. Dispenser according to one of the preceding claims, characterized in that it is constructed as a one-way and/or disposable dispenser (1, 1a) for the dispensing of a fixed stored quantity of a pulverulent or liquid medium in partial strokes or one passage of a single overall pump stroke, particularly with an additional feed medium and that the stored quantity is preferably released exclusively by opening a medium chamber (12, 12a).

## Revendications

1. Distributeur de produits (1, 1a), comprenant un corps de base (2, 2a), au moins une pompe (6, 6a) qui peut être actionnée manuellement et qui comprend une tige d'actionnement (19, 19a), au moins une chambre de pompe (9, 9a), au moins un canal de sortie (11, 11a, 18a) conduisant à une ouverture distributrice (14, 14a), au moins une chambre (12, 12a) destinée au produit liquide et/ou pulvérulent, une unité à piston qui peut être déplacée manuellement par rapport à la chambre destinée au produit (12, 12a) et qui comprend au moins un élément constitutif (8, 22, 8a, 22a) analogue à un piston, lequel doit être déplacé avec la tige d'actionnement (19, 19a), et comprenant une pièce de remplissage (21, 44) qui constitue un organe de fermeture de la chambre destinée au produit (12, 12a) et une délimitation du canal de sortie (11, 11a, 18a), cependant qu'au moins un élément constitutif analogue à un piston (8, 22, 8a, 22a) est disposé sur la pièce de remplissage (21, 44) par enfoncement, caractérisé par le fait que la tige d'actionnement (19, 19a) est prévue pour l'actionnement de la pompe (6, 6a), et par le fait que la tige d'actionnement (19, 19a) entoure le canal de sortie (11, 11a, 18a).

2. Distributeur selon la revendication 1, caractérisé par le fait que la chambre destinée au produit (12, 12a) constitue, dans au moins une position du distributeur (1, 1a), une partie à section transversale élargie du canal de sortie (11, 11a) qui conduit de la chambre de pompe (9, 9a) à l'ouverture distributrice (14, 14a), par le fait qu'en particulier, au moins une chambre destinée au produit (12, 12a) est prévue en étant séparée de la chambre de pompe correspondante (9, 9a), par le fait que, de préférence, au moins une chambre destinée au produit (12, 12a) communique avec l'ouverture distributrice (14, 14a) et/ou avec la chambre de pompe (9, 9a) dans au moins une position du distributeur (1, 1a) ou, respectivement, qu'elle est fermée pour l'essentiel d'une manière complètement étanche dans au moins une autre position du distributeur (1, 1a), et par le fait qu'en particulier, la pièce de remplissage (21, 44) traverse la chambre destinée au produit (12, 12a) ou, respectivement, qu'elle est disposée d'une manière mobile dans la chambre destinée au produit (12, 12a).

3. Distributeur selon la revendication 1 ou 2, caractérisé par le fait que la chambre destinée au produit (12, 12a) est située pour l'essentiel d'une manière coaxiale par rapport à la pompe (6, 6a), au canal de sortie (11, 11a) ou à l'ouverture de sortie (14, 14a), en étant plus proche de l'ouverture distributrice (14, 14a) que la pompe (6, 6a), en étant située à l'intérieur d'au moins une enveloppe (3, 5a) qui l'entoure à distance, en étant raccordée à au moins une soupape (16, 18 , 16a) ou à une fermeture en forme de chicane (18a), respectivement, en étant en communication directe avec la pompe (6, 6a), en étant au voisinage immédiat de l'ouverture distributrice (14) ou de la pompe (6a), respectivement, en étant raccordée à au moins une partie en forme de fente d'un canal (11, 11a) et/ou en étant coaxiale par rapport à un axe central (10, 10a) du distributeur, et par le fait qu'en particulier, un canal en forme de chicane (18a) qui arrive dans le canal de sortie (11a) est formé entre la pièce de remplissage (44) et un piston (22a) sur le pourtour extérieur de la pièce de remplissage (44) et/ou qu'un mandrin de fermeture (52) est enfoncé dans le canal de sortie (11a).

4. Distributeur selon l'une des revendications précédentes, caractérisé par le fait qu'une sortie (17) et/ou une entrée (15, 15a) de la chambre destinée au produit (12, 12a) est commandée par une soupape (18, 16, respectivement 16a), et en particulier par une soupape à tiroir qui peut être actionnée par l'organe d'actionnement manuel destiné a la pompe (6, 6a), par le fait que, de préférence, des soupapes (16, 18) qui peuvent être actionnées simultanément sont prévues dans la région d'extrémités opposées de la chambre destinée au produit (12), et par le fait qu'en particulier, au moins un corps de soupape (22) est constitué par un couvercle frontal analogue à un piston qui correspond à peu près à la cote de passage de la chambre destinée au produit (12) et/ou par le fait qu'un corps de soupape (22) est prévu sur la pièce de remplissage (21) ou qu'il est formé par la pièce de remplissage (21), respectivement, par le fait qu'en particulier, un corps de soupape (22) est réalisé sous la forme d'un corps annulaire, et par le fait que, de préférence, une partie de canal (23) débouchant dans la surface frontale du piston d'un élément constitutif (8) analogue à un piston se raccorde à l'extrémité arrière d'une partie de canal (25) qui est délimitée par une partie de tige de la pièce de remplissage (21) et par un prolongement porteur (19) destiné à un élément constitutif (8) analogue à un piston.

5. Distributeur selon l'une des revendications précédentes, caractérisé par le fait que, pour l'essentiel, la chambre destinée au produit (12a) est délimitée sur son pourtour extérieur par une enveloppe formant cylindre (7a) de la pompe (6a), par le fait qu'en particulier, la chambre destinée au produit (12a) est séparée de la chambre de pompe (9a), pour l'essentiel exclusivement par un piston de pompe (8a), et par le fait que, de préférence, le piston de pompe (8a) forme une soupape d'entrée (16a) par des fentes de contournement (45) ménagées dans la paroi du cylindre servant de corps de soupape et/ou par le fait qu'un prolongement tubulaire se raccorde à l'extrémité arrière de la chambre destinée au produit (12), par le fait qu'en particulier, une pompe (6) à piston de poussée est disposée dans un col (3) du corps de base (2) qui est librement en saillie et qui forme l'ouverture distributrice (14) et une tubulure (4), et par le fait que, de préférence, la chambre destinée au produit (12) est située au voisinage de l'extrémité arrière de la tubulure (4).

6. Distributeur selon l'une des revendications précédentes, caractérisé par le fait qu'il est prévu un montage coulissant de la chambre destinée au produit (12a), en particulier avec une course de pompage, par le fait que, de préférence, la chambre destinée au produit (12a) est délimitée à des extrémités opposées par des pistons (8a, 22a) qui peuvent coulisser le long d'une surface latérale de chambre ou d'un cylindre de pompe, respectivement, et par le fait qu'en particulier, il est prévu une succession (26) d'opérations d'actionnement de la pompe (6, 6a) et d'ouverture de la chambre destinée au produit (12, 12a), ou, respectivement, qu'il est prévu une étape de compression préalable pour la chambre de pompe (9) avant l'ouverture de la chambre de pompe (9) vers la chambre destinée au produit (12) et/ou par le fait qu'une pompe (6) présente une enveloppe de cylindre (7, respectivement 7a) qui est d'un seul tenant et en forme de godet et, respectivement, qui est ouverte vers l'extrémité arrière du fond et qui est fermée le cas échéant après le remplissage par du produit ou, respectivement, après la mise en place d'un élément constitutif (22a) analogue à un piston, par le fait qu'en particulier, une enveloppe de cylindre (7, 7a) ou un piston de pompe (8), respectivement, est fixé au corps de base (2, 2a) par l'intermédiaire d'une liaison réalisée uniquement par enfoncement, et par le fait que, de préférence, le corps de base (2, 2a) constitue par une surface d'extrémité arrière (33, 33a) une enveloppe (5, 5a) qui reçoit l'enveloppe de cylindre (7, 7a) et par rapport à laquelle la surface extérieure (34, 34a) d'un fond de l'enveloppe de cylindre (7, 7a) est en particulier décalée vers l'arrière dans la position de départ.

7. Distributeur selon l'une des revendications précédentes, caractérisé par le fait que l'actionnement de la pompe (6, 6a) est bloqué dans une position de départ prévue pour le déclenchement de la course de la pompe, en particulier avec un encliquetage élastique, et ce blocage pouvant être supprimé à force, et par le fait que, de préférence, il est prévu une ouverture de la chambre destinée au produit (12, 12a) par actionnement de la pompe (6, 6a), au moins une soupape (16, 18) prévue pour la chambre destinée au produit (12) pouvant être actionnée ou, respectivement, ouverte à peu près au début d'un entraînement de la pompe, par le fait qu'en particulier, la pièce de remplissage (21) est montée sur un col (19) de l'enveloppe d'un corps de base (2) en étant délimitée par une butée ou, respectivement, qu'un piston (8) est bloqué par rapport à un organe d'encliquetage (30) d'une paroi de cylindre, et/ou par le fait qu'en vue de l'ouverture préalable d'une sortie (17) de la chambre destinée au produit (12) et du début immédiatement consécutif du fonctionnement de la pompe, une surface d'extrémité avant d'un prolongement porteur (19) destiné à un piston de pompe (8) forme une surface d'entraînement (27) qui est opposée à une surface formant butée (28) du piston de pompe (8) à une distance égale à un trajet d'ouverture de la soupape d'une soupape de sortie (18), par le fait qu'en particulier, le piston de pompe (8) pénètre dans un pourtour intérieur d'une enveloppe de cylindre (7) pour l'entraînement sur le trajet d'ouverture par l'intermédiaire d'un blocage, et par le fait que, de préférence, un blocage en position d'au moins un élément constitutif (8, 22) analogue à un piston présente des organes d'encliquetage (30, 31) qui viennent en saillie mutuellement, comme une partie en saillie d'encliquetage (30) sur l'enveloppe de cylindre (7) et une rainure d'encliquetage (31).

8. Distributeur selon l'une des revendications précédentes, caractérisé par le fait que la chambre destinée au produit (12, 12a) et/ou une entrée (15, 15a) de la chambre destinée au produit (12, 12a) présentent en section transversale un agencement en forme de couronne ou d'anneau, respectivement, en étant en particulier coaxiales, par le fait que, de préférence, le centre de la chambre destinée au produit (12, 12a) ou de l'entrée (15, 15a), respectivement, est traversé par une tige, comme une tige de piston (20), et/ou par le fait que le produit arrive, lors de la distribution, depuis la chambre destinée au produit (12, 12a) jusque dans un canal de sortie (11a) à travers une sortie (17a), puis à travers l'ouverture de sortie (14, 14a), par le fait qu'en particulier, le canal de sortie (11) est délimité en présentant une forme annulaire, et par le fait que, de préférence, le produit est refoulé lors de sa distribution depuis une extrémité de la chambre destinée au produit (12, 12a) vers l'extrémité opposée.

9. Distributeur selon l'une des revendications précédentes, caractérisé par le fait qu'au moins un dispositif impartissant un tourbillonnement au produit est associé au moins à la sortie (17, 17a) de la chambre destinée au produit (12, 12a) ou à l'ouverture distributrice (14, 14a), respectivement, par le fait qu'en particulier, une surface d'impact (22) ou similaire est prévue dans une chambre de tourbillonnement ou de distribution (13) qui constitue l'ouverture de sortie (14, 14a), et par le fait que, de préférence, la surface d'impact (22) ou similaire peut être retirée de la chambre destinée au produit (12) ou, respectivement, qu'elle est constituée par un couvercle de fermeture prévu pour la chambre destinée au produit (12), et/ou par le fait qu'un canal en forme de chicane (18a) est raccordé directement à la sortie (17a), par le fait qu'en particulier, le canal en forme de chicane (18a) part d'une surface frontale arrière d'un élément constitutif (22a) analogue à un piston qui ferme la chambre destinée au produit (12a), et par le fait que, de préférence, le canal en forme de chicane (18a) est adapté à la capacité d'écoulement du produit en ce qui concerne sa largeur pour que le produit ne passe qu'en présence de la pression de fonctionnement.

10. Distributeur selon l'une des revendications précédentes, caractérisé par le fait que la chambre destinée au produit (12, 12a) est prévue pour servir d'accumulateur de produit pulvérulent ou liquide, respectivement, par le fait qu'en particulier, la chambre destinée au produit (12, 12a) et la chambre de pompe (9, 9a) sont prévues pour des produits différents ou, respectivement, que la chambre de pompe (9, 9a) est prévue sous la forme d'une chambre de pompe qui est dépourvue d'entrée, qui est fermée à l'opposé du piston de pompe (8, 8a) et qui constitue un organe d'actionnement manuel (34, 34a), et par le fait que, de préférence, l'ouverture distributrice (14, 14a) présente une cote de passage qui est supérieure à celle du canel de sortie (11, 11a), de la chambre destinée au produit (12, 12a) et/ou de la chambre de pompe (9a).

11. Distributeur selon l'une des revendications précédentes, caractérisé par le fait qu'il est prévu au moins un dispositif (48, 49) pour maintenir au sec ou similaire au moins un produit accumulé, des surfaces de guidege du produit du dispositif ou similaires, cependant qu'il est prévu de préférence au moins une chambre remplie d'un agent de dessiccation.

12. Distributeur selon l'une des revendications précédentes, caractérisé par le fait que la pompe (6, 6a) ou la chambre destinée au produit (12, 12a), respectivement, est située pour l'essentiel complètement à l'intérieur d'une enveloppe (5, 5a) du corps de base (2, 2a) dans eu moins une position, et en particulier dès la position de départ, enveloppe dans laquelle elle pénètre à peu près dans la direction longitudinale d'une tubulure distributrice (4, 4a), et par le fait que, de préférence, la pompe (6, 6a) et la chambre destinée au produit (12, 12a), respectivement, sont reliées entre elles par construction sous la forme d'une unité par l'intermédiaire d'au moins une liaison par enfoncement ou, respectivement, qu'elles sont reliées au corps de base (2, 2a) qui constitue en particulier une enveloppe extérieure (5, 5a).

13. Distributeur selon l'une des revendications précédentes, caractérisé par le fait qu'une tige de la pompe (6, 6a), comme par exemple une tige de piston unique (20, 20a), est introduite dans un prolongement porteur (19, 19a) du corps de base (2, 2a) qui constitue la tige d'actionnement, par le fait qu'en particulier, le prolongement porteur tubulaire (19, 19a) qui entoure la chambre destinée au produit (12) est situé pour l'essentiel sans contact à l'intérieur d'un col (3, 3a) ou, respectivement, partiellement dans une enveloppe (5, 5a) du corps de base (2, 2a), et par le fait que, de préférence, le prolongement porteur (19, 19a) se prolonge d'un seul tenant à son extrémité avant par un col (3, 3a) qui est librement en seillie et qui constitue par son extrémité une tubulure distributrice (4, 4a), et/ou par le fait qu'une tige de piston (20, 20a) qui présente la pièce de remplissage (21, 44) est enfoncée dans un prolongement porteur (19, 19a) du corps de base (2, 2a), par le fait qu'en particulier, une tige de piston (20, 20a) est introduite d'une manière étanche dans le prolongement porteur (19, 19a), et par le fait que, de préférence, un piston de pompe (8, 8a) porte à la fin de la course de pompage sur le fond de la chambre de pompe (9, 9a) ou, respectivement, que l'enveloppe de cylindre (7, 7a) est située pour sa majeure partie à l'intérieur du col (3, 3a).

14. Distributeur selon l'une des revendications précédentes, caractérisé par le fait qu'un piston de pompe (8) constitue par construction une unité avec un corps de soupape mobile (22) d'une soupape de sortie (18) de la chambre destinée au produit (12), par le fait qu'en particulier, l'unité obtenue par construction est exclusivement disposée par liaison par enfoncement sur un prolongement porteur (19) du corps de base (2), et par le fait que, de préférence, un blocage axial de l'unité obtenue par construction est constitué par un appui sans jeu du corps de soupape (22) sur le produit contenu dans la chambre destinée au produit (12) et/ou par le fait qu'une enveloppe de cylindre (7a) qui constitue la chambre destinée au produit (12a) est librement portée d'une manière approximativement exclusive par venue en prise dans le pourtour extérieur d'un piston de pompe annulaire (8a), par le fait qu'en particulier, une unité à piston composée d'un piston de pompe (8) et d'une tige de piston (20) comporte un corps de soupape (22) enfoncé séparément, et par le fait que, de préférence, la tige de piston (20) est reçue pour l'essentiel complètement dans un prolongement porteur (19) du corps de base (2) par venue en prise par enfoncement.

15. Distributeur selon l'une des revendications précédentes, caractérisé par le fait qu'une enveloppe (5, 5a) du distributeur (1, 1a) - vue dans la direction d'un axe central (10, 10a) - est plate et allongée, et qu'elle est pourvue en particulier de deux côtés opposés plus plats (37), par le fait que, de préférence, l'un au moins des côtés plus plats (37) est traversé par un évidement de venue en prise (36) pour former un endroit de venue en prise des doigts, et par le fait qu'en particulier, il est prévu, en plus des côtés plus plats (37), une autre surface de pose (33) destinée à disposer le distributeur (1, 1a) sur une surface d'appui continue d'une manière moins stable que sur les côtés plus plats (37), ou, respectivement, que l'autre surface de pose (33) est constituée par une surface d'extrémité arrière du corps de base (2, 2a), et/ou par le fait que le corps de base (2, 2a) forme une surface d'extrémité arrière (33, 33a) qui est divisée en deux parties, par le fait qu'en particulier, une enveloppe de cylindre (7a) qui forme la chambre destinée au produit (12a) est située à distance pour l'essentiel entre les surfaces partielles de la surface d'extrémité (33a) lorsqu'elle est vue de côté, et par le fait que, de préférence, la distance entre deux évidements opposés (36) ménagés dans une surface latérale du corps de base (2) est inférieure à leur largeur ou, respectivement, que cette largeur est supérieure au diamètre de l'enveloppe de cylindre (7a).

16. Distributeur selon l'une des revendications précédentes, caractérisé par le fait que deux parois plates (37) de la surface latérale d'une enveloppe (5) du corps de base (2) se prolongent mutuellement par l'intermédiaire de bords latéraux (38) qui sont fortement courbés dans le sens convexe, par le fait qu'en particulier, l'enveloppe (5), lorsqu'elle est vue dans le sens axial, fait saillie de tous les côtés au-delà du pourtour extérieur d'un col (3) du corps de base (2) qui constitue une tubulure distributrice (4), et par le fait que, de préférence, les bords latéraux (38) se raccordent à des surfaces partielles d'une surface d'extrémité arrière (33) de l'enveloppe (5) qui présentent approximativement la forme d'un U ou d'un V, respectivement, pourvu d'ailes dirigées l'une vers l'autre, et/ou par le fait que les surfaces partielles (33) sont situées pour l'essentiel dans l'alignement d'organes d'actionnement manuel (35) du corps de base (2), par le fait qu'en particulier, le distributeur (1) présente pour l'essentiel la forme d'un T ou d'un Y, respectivement, en vue de côté, et par le fait que, de préférence, l'aile transversale du T de la forme en T constitue des extrémités qui sont coudées en s'éloignant de la semelle du T et qui font librement saillie.

17. Distributeur selon l'une des revendications précédentes, caractérisé par le fait qu'il est prévu des organes d'actionnement manuel qui sont dirigés à l'opposé les uns des autres et qui constituent des surfaces d'appui des doigts (34, 35, respectivement 34a, 35a), par le fait que, de préférence, deux organes d'actionnement manuel présentant la même direction de poussée sont constitués des deux côtés d'une tubulure distributrice (4, 4a) par une paroi frontale d'une enveloppe extérieure (5, 5a) ou, respectivement, qu'un organe d'actionnement manuel (34) est constitué par un tiroir (7) qui est situé pour l'essentiel à l'intérieur de l'enveloppe (5, 5a), et/ou par le fait que le distributeur est réalisé en vue d'être porté et actionné simultanément d'une seule main, par le fait qu'en particulier, deux organes d'actionnement manuel (35, 35a) sont constitués par des parties d'extrémité d'une paroi frontale allongée du corps de base (2, 2a), et par le fait que, de préférence, le distributeur (1, 1a) est plat et allongé en vue axiale.

18. Distributeur selon l'une des revendications précédentes, caractérisé par le fait qu'il est prévu pour l'ouverture distributrice (14, 14a) un couvercle amovible (42, 42a), en particulier en forme de capuchon, et par le fait que, de préférence, le couvercle (42, 42a) comporte un organe de fermeture (52) en saillie sur son côté intérieur, comme un mandrin sur une paroi frontale en vue de sa venue en prise directe dans une entrée plus étroite qu'une chambre distributrice (13a), et/ou par le fait qu'un couvercle (42, 42a) est enfoncé sur le pourtour extérieur d'une tubulure (4) du corps de base (2), par le fait qu'en particulier, le pourtour extérieur est en retrait, et par le fait que, de préférence, le couvercle (42, 42a) porte sur une surface frontale ou, respectivement, qu'il se prolonge par un col (3) qui forme la tubulure (4) en vue de son blocage à l'encontre d'un enlèvement accidentel.

19. Distributeur selon l'une des revendications précédentes, caractérisé par le fait qu'il est réalisé sous la forme d'un distributeur jetable ou à usage unique (1, 1a) en vue de la distribution d'une quantité accumulée déterminée d'un produit pulvérulent ou liquide, respectivement, au cours de trajets partiels ou d'un parcours complet, respectivement, sur une course de pompage d'ensemble unique, en particulier au moyen d'un agent de déplacement supplémentaire, et par le fait que la quantité accumulée est de préférence libérée exclusivement par l'ouverture d'une chambre destinée au produit (12, 12a).
